## Europäisches Patentamt

(19) **European Patent Office**

**Office européen des brevets**

(11) Veröffentlichungsnummer: **0 058 767**

**B2**

(12) # NEUE EUROPÄISCHE PATENTSCHRIFT

(45) Veröffentlichungstag der neuen Patentschrift:
15.11.89

(21) Anmeldenummer: **81109694.0**

(22) Anmeldetag: **14.11.81**

(51) Int. Cl.⁴: **C 08 F 220/54,** C 08 F 220/32,
C 12 N 11/08 //
(C08F220/54, 220:32),
(C08F220/32, 220:54)

(54) **Verfahren zur Herstellung von perlförmigen, hydrophilen, gegenüber Proteinen bindungsaktiven Trägerpolymeren.**

(30) Priorität: **21.02.81 DE 3106456**

(43) Veröffentlichungstag der Anmeldung:
**01.09.82 Patentblatt 82/35**

(45) Bekanntmachung des Hinweises auf die Patenterteilung:
**30.01.85 Patentblatt 85/5**

(45) Bekanntmachung des Hinweises auf die Entscheidung über den Einspruch:
**15.11.89 Patentblatt 89/46**

(84) Benannte Vertragsstaaten:
**CH DE FR GB LI SE**

(73) Patentinhaber: **Röhm GmbH, Kirschenallee Postfach 4242, D-6100 Darmstadt 1 (DE)**

(72) Erfinder: **Krämer, Dieter, Dr., An der Favorite 4, D-6500 Mainz (DE)**
Erfinder: **Pennewiss, Horst, Dr., Meissnerweg 53, D-6100 Da- Neu- Kranichstein (DE)**
Erfinder: **Plainer, Hermann, Dr., Am Wembach 15, D-6107 Reinheim 1 (DE)**
Erfinder: **Schnee, Reiner, Dr., Arheilger Woogstrasse 62, Darmstadt- Arheilgen (DE)**
Erfinder: **Schleier, Waldemar, Gutenbergstrasse 42, D-6100 Darmstadt (DE)**

(56) Entgegenhaltungen:
**DE-A-2 237 316**
**DE-A-2 343 633**
**DE-A-2 722 751**
**FR-A-2 442 248**
**GB-A-1 483 587**

**Makromol. Chem. 176 (1975) SS. 657-676**

EP 0 058 767 B2

**Beschreibung**

Die Erfindung betrifft ein Verfahren zur Herstellung von perlförmigen, hydrophilen, gegenüber Proteinen bindungsaktiven Trägerpolymeren gemäß dem Oberbegriff des Hauptanspruches.

Aus der DE-AS-2 237 316 ist es bereits bekannt, perlförmige, vernetzte, in Wasser quellbare Mischpolymerisate durch inverse Perpolymerisation eines Monomerengemisches aus einem gegenüber Proteinen bindungsaktiven Monomeren, einem vernetzenden Monomeren und einem hydrophilen Monomeren herzustellen. Um eine gute Quellbarkeit und eine weitmaschige Vernetzung zu erreichen, wird das Monomerengemisch in einem Lösungsmittel, das mit dem unpolaren organischen Polymerisationsmedium nicht mischbar ist, gelöst, die Lösung in diesem Medium zu Tröpfchen suspendiert und in dieser Form polymerisiert. Als Lösungsmittel für das Monomerengemisch werden z. B. Formamid, Dimethylformamid oder Dimethylsulfoxid verwendet.

Gemäß DE-OS-2 722 751 wird die Bindungsaktivität der auf diesem Wege hergestellten hydrophilen Perlpolymerisate noch erhöht, wenn der Anteil der vernetzenden Monomeren am Monomerengemisch 5 Gew.-% oder mehr beträgt und der Gewichtsanteil an Acryl- oder Methacrylamid oder an Methylen-bis-acrylamid oder -methacrylamid bestimmte Grenzwerte übersteigt. Durch dieses Verhalten entstehen Polymerisate mit einer Hohlperlstruktur, die als wesentlich für die hohe Bindungsaktivität angesehen wurde.

Die bekannten hydrophilen Perlpolymerisate enthalten als gegenüber Proteinen bindungsaktive Gruppen Monomereinheiten mit Oxirangruppen (Epoxgruppen), z. B. Glycidylmethacrylat oder Allylglycidyläther. Diese Einheiten vermögen wasserlösliche Proteine covalent unter Erhaltung ihrer biologischen Aktivität an das Polymerisat zu binden. Obwohl auf diese Weise mit einigen Enzymen hohe Bindungskapazitäten erreicht werden konnten, blieb die Bindungskapazität bei einigen anderen Enzymen unbefriedigend. Unter der Bindungskapazität wird diejenige enzymatische Aktivität verstanden, die sich bei maximaler Beladung des Perlpolymerisats mit einem bestimmten Enzym erreichen läßt. Es hat sich gezeigt, daß sich die Bindungskapazität oberhalb einer enzymspezifischen Grenze nicht oder nicht wesentlich dadurch erhöhen läßt, daß man den Oxirangruppengehalt des Perlpolymerisats erhöht oder eine Enzymlösung von höherer Konzentration zur Beladung einsetzt.

Der Erfindung liegt die Aufgabe zugrunde, die Bindungskapazität von hydrophilen Perlpolymerisaten, die Proteine covalent an Oxirangruppen binden, zu erhöhen. Im engeren Sinne besteht die Aufgabe darin, die Bindungskapazität eines perlförmigen Trägerpolymerisats in Bezug auf das jeweils zu bindende Protein zu optimieren.

Es wurde nun gefunden, daß bei dem Verfahren gemäß Anspruch Perlpolymerisate mit erhöhter und gegebenenfalls für bestimmte Enzyme optimierter Bindungskapazität entstehen. Die Bindungskapazität wird erfindungsgemäß durch den Zusatz einer speziellen Kombination von Verdünnungsmitteln zu dem Monomerengemisch eingestellt.

Als Komponente A des Verdünnungsmittels können Wasser, Formamid, Glykol und Dimethylsulfoxid oder deren Gemische eingesetzt werden. Überraschenderweise bleibt die Bindungsfähigkeit für Proteine auch bei Verwendung von Wasser erhalten, obwohl Wasser mit den zur Proteinbindung erforderlichen Epoxidgruppen leicht reagiert.

Als Komponente B des Verdünnungsmittels erwiesen sich organische Flüssigkeiten von mittlerer bis hoher, durch Sauerstoffatome vermittelter Polarität als geeignet. Eine ausreichende Polarität ist gegeben, wenn der Gehalt an Sauerstoff wenigstens 20 Gew.-% beträgt und das Molekulargewicht unter 200 liegt. Bevorzugt sind aliphatische organische Flüssigkeiten, die Sauerstoff in Form von Carbonylgruppen oder Hydroxylgruppen enthalten; dagegen sind Carboxyl-Gruppen wegen ihrer hohen Reaktivität gegenüber Oxirangruppen weniger bevorzugt. Auch ätherartig gebundene Sauerstoffatome tragen zu der erwünschten Polarität bei und finden sich beispielsweise in aliphatischen oder Cycloaliphatischen Äthern und aliphatischen Estern. Die für das Verfahren der Erfindung geeigneten Komponenten B zeichnen sich über wiegend durch ein Gesamt-Löslichkeitsparameter $\delta$ von 9 bis 15 und bevorzugt 11 bis 14,5 Hildebrand-Einheiten oder durch einen dispersiven Löslichkeitsparameter $\delta_D$ im Bereich von 7 bis 9, vorzugsweise 7,4 bis 8,5 Hildebrand-Einheiten aus (vgl. Polymer-handbook, 2. Ausgabe, 1975, S. IV 337 ff.)

Die nachfolgende Liste enthält Beispiele der Verdünnungsmittelkomponente B und ihre Kenngrößen:

| | Molekular-gewicht | Sauerstoff-gehalt (Gew.-%) | Löslichkeits-parameter | |
| --- | --- | --- | --- | --- |
| | | | $\delta$ | $\delta_D$ |
| Methanol | 32 | 50,0 | 14,3 | 7,4 |
| Äthanol | 46 | 35,0 | 12,9 | 7,7 |
| n-Propanol | 60 | 26,7 | 11,9 | 7,8 |
| i-Propanol | 60 | 26,7 | 11,5 | - |
| n-Butanol | 74 | 21,6 | 11,3 | 7,8 |
| Aceton | 58 | 27,6 | 9,8 | 7,6 |
| Diacetonalkohol | 116 | 27,6 | 10,2 | 7,7 |
| Methyläthylketon | 72 | 22,2 | 9,3 | 7,8 |
| Tetrahydrofuran | 72 | 22,2 | 9,5 | 8,2 |

| | | | | |
|---|---|---|---|---|
| Dimethylformamid | 73 | 22,0 | 12,1 | 8,5 |
| Äthylacetat | 88 | 36,4 | 9,1 | 7,4 |
| Äthylglykol | 90 | 35,5 | 8,3 | - |
| Diäthylglykol | 118 | 27,1 | 11,9 | 7,9 |

Die Mischung aus den Monomeren und den Verdünnungsmittelkomponenten A und B muß, möglichst schon bei Raumtemperatur (20°C), eine homogene Phase bilden, was nicht bei allen denkbaren Kombinationen der Fall ist. Weiterhin muß diese Mischung wenigstens bei der Polymerisationstemperatur mit dem organischen Medium unverträglich sein, so daß sich eine getrennte suspendierte Monomerenphase ausbildet. Wenn die Verdünnungsmittelkomponenten in dem organischen Medium eine gewisse Löslichkeit haben, kann die Monomerenphase daran verarmen, was eine Änderung der Bindungskapazität nach sich ziehen kann. Um die Bindungskapazität dennoch gezielt einstellen zu können, empfiehlt es sich, das organische Medium vorher mit der darin löslichen Verdünnungsmittelkomponente A bzw. B zu sättigen bzw. die entsprechende Gleichgewichtskonzentration einzustellen. Dadurch steigt die Polarität des organischen Mediums an. Der Zusatz der Verdünnungsmittelkomponenten zu dem organischen medium muß unter der Grenze bleiben, bei der sich die Monomerenphase in dem organischen Medium lösen würde.

Durch die Wahl eines ausreichend unpolaren organischen Mediums kann die Annäherung an diese Grenze vermieden werden. Bevorzugt sind aliphatische Kohlenwasserstoffe und Chlorkohlenwasserstoffe und deren Mischungen.

Es ist von Vorteil, wenn die Komponenten A und B, möglichst schon bei Raumtemperatur, miteinander vollständig mischbar sind. Eine bevorzugte Gruppe aus den organischen Flüssigkeiten der Komponente B sind die bei Raumtemperatur mit Wasser vollständig mischbaren Flüssigkeiten, wie Methanol, Äthanol, Propanol, Aceton, Dimethylformamid oder Tetrahydrofuran. Eine auch unter technischen Gesichtspunkten besonders vorteilhafte Kombination ist das Gemisch aus Methanol und Wasser.

Das Mischungsverhältnis der Komponenten A und B untereinander kann in weiten Grenzen, etwa zwischen 99 : 1 bis 1 : 99 Gewichtsteilen, gewählt werden, wobei der Bereich von 90 : 10 bis 10 : 90 bevorzugt ist. Der Anteil des Verdünnungsmittels, bestehend aus A und B, an der Monomerenphase kann zwischen 20 und 90 Gew.-% liegen und beträgt vorzugsweise 50 bis 90 Gew.-%. Zur Festlegung dieses Anteils und des Verhältnisses von A zu B geht man zweckmäßig so vor, daß man eine Reihe verschiedener Perlpolymerisate in Versuchsansätzen herstellt und jeweils die Bindungskapazität für ein bestimmtes Protein ermittelt. Dabei findet man im Regelfall bei der Variation des Verhältnisses A : B einen Anstieg der Bindungskapazität bis zu einem Höchstwert und danach ein Absinken, wie nachfolgend am Beispiel der inversen perlpolymerisation einer Monomerenphase aus

30 Gew.-T. Methacrylamid
30 Gew.-T. N,N'-Methylen-bis-methacrylamid
20 Gew.-T. Glycidylmethacrylat
20 Gew.-T. Allyl-Glycidyläther
73 Gew.-T. eines Methanol-Formamid-Gemisches

bei der Optimierung an PC-Acylase gezeigt wird: PC-Acylase = Penicillin-Acylase

| Verdünnungsmittel | | | Maximale Enzymaktivität (U/g) |
|---|---|---|---|
| A | | B | |
| Formamid | | Methanol | |
| 10 | : | 90 | 71 |
| 30 | : | 70 | 101 |
| 50 | : | 50 | 124 |
| 90 | : | 10 | 125 |
| 100 | : | 0 | 95 |

Für andere Enzyme kann das Aktivitätsmaximum bei einem anderen Verhältnis A : B liegen.

Um die Hydrophilie des Perlpolymerisats zu gewährleisten, muß das zugrundeliegende Monomerengemisch zum überwiegenden Teil aus hydrophilen Monomeren bestehen. Dazu gehören Acryl- und Methacrylamid und ihre Methylen-bis-amide sowie solche ungesättigten, radikalisch polymerisierbaren Monomeren, die bei Raumtemperatur wenigstens 10 prozentige wäßrige Lösungen bilden und vorzugsweise keine ionischen oder durch Säure- oder Basenzusatz ionisierbaren Gruppen enthalten. Beispiele bevorzugter hydrophiler Comonomerer sind Hydroxyalkylester von ungesättigten polymerisierbaren Carbonsäuren, wie Hydroxyäthylacrylat und -methacrylat, 2-Hydroxypropylacrylat oder -methacrylat, und N-Vinylpyrrolidon. Acryl- oder Methacrylamid und ihre Methylen-bis-amide machen wenigstens 30 Gew.-% und vorzugsweise wenigstens 50 Gew.-% aus. Weitere hydrophile Comonomere können gegebenenfalls bis zu 65 Gew.-% der polymerisierbaren Monomeren ausmachen.

Monomere mit Oxirangruppen sind an dem Monomerengemisch in Mengen von 5 bis 60 Gew.-% beteiligt. Beispiele dieser Monomeren sind Glycidyl-acrylat und -methacrylat und Allylglycidyläther, die gegebenenfalls auch gemeinsam verwendet werden können. Nach der Umsetzung des Perlpolymerisats mit einem Protein und fortdauernder Berührung mit einem wäßrigen Medium gehen die Oxirangruppen allmählich vollständig in Hydroxygruppen über, wodurch die Hydrophilie des Polymerisats weiter ansteigt. Weitere Comonomere, die weniger hydrophil sind und bei Raumtemperatur weniger als 10 prozentige gesättigte wäßrige Lösungen ergeben, vermindern die Hydrophilie des Polymeren und werden, falls überhaupt, in Mengen unter 25 Gew.-% eingesetzt.

Die erfindungsgemäß hergestellten Perlpolymerisate sind stark vernetzt. Die als Vernetzungsmittel wirksamen Comonomeren mit zwei oder mehr äthylenisch ungesättigten, polymerisierbaren Gruppen im Molekül machen wenigstens 5 Gew.-% des Monomerengemisches aus. Vorzugsweise dienen Methylenbisacrylamid oder -methacrylamid als Vernetzungsmittel. In diesem Falle kann der Anteil der vernetzenden Monomeren 90 Gew.-% der polymerisierbaren Monomeren übersteigen.

Als weitere Bestandteile enthält die suspendierte Monomerenphase Polymerisationsinitiatoren und gegebenenfalls weiter Hilfsmittel, wie aus dem einschlägigen Stand der Technik bekannt ist.

Das Verfahren der inversen Perlpolymerisation ist an sich bekannt. Man suspendiert die Monomerenphase unter Rühren in einem organischen Medium, das vorzugsweise ein darin lösliches Dispergiermittel enthält, zu Tröpfchen von etwa 10 bis 1 000 µm Größe. Die Größe der Tröpfchen läßt sich durch die Wahl von Art und Menge des Dispergiermittels und der Rührgeschwindigkeit auf den gewünschten Wert einstellen. Die Polymerisation findet innerhalb der Monomertröpfchen statt und wandelt sie in feste Perlpolymerisate um, die nach Abschluß der Polymerisation von dem organischen Medium abgetrennt werden können. Vorteilhafte Ausführungsformen dieses Verfahrens sind in der DE-PS-2 009 218, der DE-AS-2 237 316 und der DE-OS-2 722 751 beschrieben.

In manchen Fällen neigen die Perlpolymerisate zum Verkleben zu größeren Aggregaten, was aus Gründen der Handhabbarkeit im allgemeinen unerwünscht ist. Dieser Nachteil läßt sich vermeiden, wenn man in der Monomerenphase ein Polymerisat löst, das ihre Viskosität erhöht. Um die Hydrophilie des Endproduktes nicht zu beeinträchtigen, sollte dieser Polymerisatzusatz ebenfalls hydrophil sein und kann beispielsweise aus den gleichen Monomeren wie das Perlpolymerisat mit Ausnahme der vernetzenden Monomeren aufgebaut sein. Im Falle der Verwendung eines Wasser-Methanolgemisches als Verdünnungsmittel eignet sich z. B. Polymethacrylamid. Wirksame Mengen derartiger Polymerisatzusätze liegen z. B. zwischen 0,1 und 20 Gew.-%, bezogen auf das Gewicht der Monomerenphase. Weitere verwendbare Polymerisatzusätze sind z. B. Mischopolymerisate von niederen Acrylsäurealkylestern, wie Butylacrylat, mit Acryl- oder Methacrylamid oder Glycidylmethacrylat.

Nach Abschluß der Perlpolymerisation lassen sich die Polymerisatperlen von dem organischen Medium abfiltrieren. Wenn als Verdünnungsmittel Wasser oder andere Verbindungen mit aktiven Wasserstoffatomen wie Alkohole oder Glykole, verwendet worden sind, müssen diese aus den Perlen entfernt werden, weil sonst bei längerer Lagerung eine Umsetzung mit den Oxirangruppen zu befürchten ist. Flüchtige Verdünnungsmittel, wie Wasser und Methanol, lassen sich leicht durch Trocknen entfernen. Schwerflüchtige Verdünnungsmittel, wie Glykol, Formamid oder Dimethylsulfoxid lassen sich durch Auswaschen mit flüchtigen Lösungsmitteln, z. B. Aceton, leicht entfernen. Anschließend werden die Reste des Lösungsmittels verdunstet. Die Perlpolymerisate sind nach dem Trocknen hart und trocken und bei Abwesenheit von Luftfeuchtigkeit beliebig lange lagerfähig.

Zur Beladung mit Proteine versetzt man die Perlen mit einer möglichst konzentrierten Lösung des Proteins, wofür praktisch nur wäßrige Lösungen in Betracht kommen. Das wichtiges Anwendungsgebiet liegt in der Immobilisierung von Enzymen, wie z. B. Pencillin-Acylase, Trypsin oder Lactase.

Die nachfolgenden Beispiele zeigen die Steigerung der Bindungskapazität durch Verwendung einer erfindungsgemäßen Verdünnungsmittelkombination (Versuch 1 Methanol/Wasser) gegenüber der Verwendung nur einer Verdünnerflüssigkeit (Versuch 2, Formamid).

Die höchste Bindungskapazität kann durch variieren des Mischungsverhältnisse der Verdünnungsmittelkomponenten A und B ermittelt werden; vgl. Versuch 9 im Beispiel III und die Tabelle in der Beschreibung. Bei einer Änderung der Polymerisatzusammensetzung kann sich die Lage des Kapazitätsmaximums in Bezug auf das Mischungsverhältnis A : B verschieben wie aus dem Vergleich des Beispiels II mit Versuch 1 hervorgeht. Dabei wird ein Vorteil eines hohen Gehalts an vernetzenden Monomeren von 30 % oder mehr, insbesondere 50 % oder mehr, bezogen auf das Gewicht des Monomerengemisches, erkennbar.

Bei gleichbleibender Polymerisatzusammensetzung kann ein Austausch einer Verdünnungsmittelkomponente durch eine andere Flüssigkeit wesentliche Aktivitätsänderungen herbeiführen, wie ein Vergleich der Versuche 6 und 7 zeigt, so daß das Maximum für jede Mischung A/B unabhängig zu ermitteln ist. Die nachfolgende Tabelle gibt eine Übersicht über die Beispiele.

| Beispiel Nr. | Versuch Nr. | Monomerengemisch (Gew.-%) | | | | Verdünnungsmittel (Gew.-%) | | | Aktivität PC-Acylase [bzw. Trypsin] |
|---|---|---|---|---|---|---|---|---|---|
| | | MA | MBMA | GM | AGE | Komp. A Wasser | Form-amid | Komp. B Methanol | |
| I | 1 | - | 80 | 10 | 10 | 20 | - | 80 | 122 |
| | 2 | - | 80 | 10 | 10 | - | 100 | - | 73 |
| | 3 | 30 | 30 | 20 | 20 | 40 | - | 60 | 0 |

4

| | | | | | | | | | |
|---|---|---|---|---|---|---|---|---|---|
| II | 4 | 30 | 30 | 20 | 20 | 80 | - | 20 | 65 [6,5] |
| | 5 | 30 | 30 | 20 | 20 | 90 | - | 10 | 7 |
| | 6 | 10 | 50 | 20 | 20 | 70 | - | 30 | 30 |
| | 7 | 10 | 50 | 20 | 20 | - | 70 | 30 | 125 |
| III | 8 | 10 | 50 | 20 | 20 | - | 10 | 90 | 71 |
| | 9 | 10 | 50 | 20 | 20 | - | 90 | 10 | 125 |
| | 10 | 10 | 50 | 20 | 20 | - | 100 | - | 95 |
| IV | 11* | 30 | 30 | 20 | 20 | 80 | - | 20 | [10,2] |

MA = Methacrylamid
MBMA = Methylen-bis-methacrylamid
GM = Glycidylmethacrylat
AGE = Allylglycidyläther
*) Mitverwendung von Polymethacrylamid in der Monomerenphase

In einem 6 l Rührkolben mit Thermometer, Wasserabscheider, Rückflußkühler, Stickstoff-Einleitungsrohr werden 1740 g n-Hexan, 1 100 g Perchloräthylen, 9 g polymerer Emulgator (Mischpolymerisat aus 95 Teilen N-Butylmethacrylat, 5 Teilen 2-Trimethylammoniumäthylmethacrylat-chlorid) und 5 g Trockeneis vorgelegt. Unter Rühren und Durchleiten von Stickstoff wird bei 50°C eine Mischung aus

640 g Methanol
160 g Wasser
240 g Methylen-bis-methacrylamid
 30 g Allylglycidyläther
 30 g Glycidylmethacrylat
  6 g 4,4'-Azobis-(4-cyanovaleriansäure)

zugegeben, in der organischen Phase verteilt und anschließend bis zum Sieden bei 65 - 70°C erhitzt. Während einem Zeitraum von ca. 6 h wird das Methanol/Wasser-Gemisch nahezu vollständig ausgekreist. Der Ansatz wird für weitere 4 Stunden zu Ende reagieren gelassen und anschließend auf Raumtemperatur abgekühlt. Die entstandenen Perlen werden abgesaugt und 12 Stunden bei 40°C im Vakuum getrocknet.

Die Aktivität nach Beladung mit PC-Acylase (wie nachfolgend beschrieben) beträgt 122 U/g.

## Kupplung mit PC-Acylase

500 mg Perlen werden mit 1,6 ml einer PC-Acylaselösung, die 1,05 ml Enzym in 0,1 m Na-acetathaltigem Kaliumphosphatpuffer von pH 7,5 enthält, 72 h bei 23°C inkubiert. Danach werden die Perlen abgesaugt, 3-mal mit 1 m Kochsalz-Lösung und 2-mal mit Phosphatpuffer pH 7,5 gewaschen. Die erhaltenen Enzymperlen werden mit 20 ml einer 2 %-igen Kalium-Penicillin G-Lösung in 0,05 m Natriumphosphatpuffer pH 7,5 bei 37°C inkubiert und die freigesetzte Phenylessigsäure mit 0,5 m NaOH titriert. Die Enzymaktivität wird auf 1 g Feuchtperlen bezogen, wobei 1 U/g einem Verbrauch von 1 µMol NaOH pro Minute und pro g Feuchtperlen entspricht.

## Beispiel 2 (Vergleichsversuch)

Apparatur und Reaktionsführung wie in Beispiel 1, mit der Ausnahme, daß kein Lösungsmittel ausgekreist wird.

## Vorlage

1 740 g n-Heptan, 1 100 g Perchloräthylen, 5,6 g Emulgator und 5 g Trockeneis.

## Monomermischung

1 200 g Formamid, 240 g Methylen-bis-methacrylamid, 30 g Allylglycidyläther, 30 g Glycidylmethacrylat, 6 g Benzoylperoxid.

Am Ende der Reaktion wird die Phase über den abgesetzten Perlen abdekantiert, die Perlen 3-mal mit je 2000 ml Aceton gewaschen und abgesaugt. Es wird noch 3-mal mit Aceton nachgewaschen und die Perlen 12 Stunden in Vakuum getrocknet. Nach der Kupplung von PC-Acylase wurde eine Aktivität von 73 U/g gefunden.

**Beispiel 3**

Apparatur, Vorlage und Reaktionsführung analog Beispiel Nr. 1.

**Monomerenphase**

480 g Methanol
320 g Wasser
90 g Methacrylamid
90 g Methylen-bis-methacrylamid
60 g Allylglycidyläther
60 g Glycidylmethacrylat
6 g 4,4'-Azobis-(4-cyanovaleriansäure)

Nach dem Versuch zur Kupplung von PC-Acylase wurde keine Enzymaktivität festgestellt.

**Beispiel 4**

Beispiel Nr. 3 wird mit 160 g Methanol und 640 g entsalztem $H_2O$ wiederholt. Man erhält Perlen, die teilweise aggregiert sind.
Nach der Kupplung von PC-Acylase wurde eine Enzymaktivität von 65 U/g gefunden.

**Beispiel 5**

Beispiel Nr. 3 wird mit 80 g Methanol und 720 g Wasser wiederholt. Nach der Kupplung von PC-Acylase wurde eine Enzymaktivität von 7 U/g gefunden.

**Beispiel 6**

Apparatur, Vorlage und Reaktionsführung analog Beispiel Nr. 1.

**Monomerenphase**

240 g Methanol
560 g Wasser
150 g Methylen-bis-methacrylamid
30 g Methacrylamid
60 g Allylglycidyläther
60 g Glycidylmethacrylat
6 g 4,4'-Azobis-(4-cyanovaleriansäure)

Nach der Kupplung von PC-Acylase wurde eine Enzymaktivität von 30 U/g gefunden.

**Beispiel 7**

Apparatur, Vorlage und Reaktionsführung analog Beispiel Nr. 2.

**Monomerenphase**

553 g Formamid
237 g Methanol
150 g Methylen-bis-methacrylamid
 60 g Allylglycidyläther
 60 g Glycidylmethacrylat
 30 g Methacrylamid
  6 g Benzoylperoxid
  6 g Dimethylanilin (wird nach Verteilung der Monomerphase in die organische Phase zugegeben)

Nach der Kupplung von PC-Acylase wurde eine Enzymaktivität von 125 U/g gefunden.

**Beispiel 8**

Beispiel Nr. 7 wird mit 79 g Formamid und 711 g Methanol wiederholt.
Nach der Kupplung von PC-Acylase wurde eine Enzymaktivität von 71 U/g gefunden.

**Beispiel 9**

Beispiel Nr. 7 wird mit 711 g Formamid und 79 g Methanol wiederholt.
Nach der Kupplung von PC-Acylase wurde eine Enzymaktivität von 125 U/g gefunden.

**Beispiel 10 (Vergleichsversuch)**

Beispiel Nr. 7 wird mit 791 g Formamid wiederholt.
Nach der Kupplung von PC-Acylase wurde eine Enzymaktivität von 95 U/g gefunden.

**Beispiel 11**

Apparatur, Vorlage und Reaktionsführung analog Beispiel Nr. 1.

**Monomerenphase**

 30 g Polymethacrylamid
160 g Methanol
640 g Wasser
 81 g Methacrylamid
 81 g N,N'-Methylen-bis-methacrylamid
 54 g Allylglycidyläther
 54 g Glycidylmethacrylat
  6 g 4,4'-Azobis-(4-cyanovaleriansäure)

Man erhält gleichmäßig runde Perlen im Gegensatz zu Beispiel Nr. 4.

# EP 0 058 767 B2

## Patentansprüche

1. Verfahren zur Herstellung eines perlförmigen vernetzten hydrophilen, gegenüber Proteinen bindungsaktiven Trägerpolymeren durch inverse Perlpolymerisation einer Monomerenphase, die als Monomere

1) 30 bis 95 Gew.-% (bezogen auf das Gesamtgewicht der polymerisierbaren Monomeren) wenigstens einer Verbindung der Gruppe Acrylamid, Methacrylamid, Methylen-bis-acrylamid und -methacrylamid,

2) gegebenenfalls weitere hydrophile, radikalisch polymerisierbare Comonomere,

3) 5 bis 60 Gew.-% wenigstens eines ungesättigten, radikalisch polymerisierbaren Monomeren mit einer Oxirangruppe,

4) gegebenenfalls bis zu 25 Gew.-% anderen radikalisch polymerisierbaren Comonomeren von geringer Hydrophilie,

und darunter wenigstens 5 Gew.-% Monomere mit wenigstens zwei polymerisierbaren Doppelbindungen sowie ein Verdünnungsmittel enthält und einem nichtwäßrigen organischen Medium zu Tröpfchen verteilt ist und in dieser Form radikalisch polymerisiert wird, dadurch gekennzeichnet, daß als Verdünnungsmittel für die Monomerenphase ein Gemisch aus

A) wenigstens einem Lösungsmittel aus der Gruppe Wasser, Formamid, Glykol und Dimethylsulfoxid und

B) wenigstens einer davon verschiedenen organischen Flüssigkeit, die mit der Mischung aus den Monomeren und der Komponente A eine homogene Phase bildet, ein Molekulargewicht unter 200 hat und wenigstens 20 Gew.-% Sauerstoff enthält, eingesetzt wird.

2. Verfahren nach Anspruch 1, dadurch gekennzeichnet, daß als Komponente B des Verdünnungsmittels eine Flüssigkeit eingesetzt wird, die den Sauerstoff in Form von Carbonyl- und/oder Hydroxylgruppe enthält.

3. Verfahren nach den Ansprüchen 1 und 2, dadurch gekennzeichnet, daß als Komponente B des Verdünnungsmittels eine organische Flüssigkeit mit einem Gesamt-Löslichkeitsparameter zwischen 9 und 15 Hildebrand-Einheiten oder einem dispersiven Löslichkeitsparameter von 7 bis 9 Hildebrand-Einheiten eingesetzt wird.

4. Verfahren nach den Ansprüchen 1 bis 3, dadurch gekennzeichnet, daß als Komponente B des Verdünnungsmittels eine bei Raumtemperatur mit Wasser vollständig mischbare Flüssigkeit eingesetzt wird.

5. Verfahren nach den Ansprüchen 1 bis 4, dadurch gekennzeichnet, daß als Verdünnungsmittel ein Gemisch aus Wasser und Methanol eingesetzt wird.

6. Verfahren nach den Ansprüchen 1 bis 5, dadurch gekennzeichnet, daß vor Beginn der Polymerisation in der Monomerenphase ein Polymeres gelöst wird.

## Claims

1. Process for preparing a head-shaped cross-linked hydrophilic carrier polymer which is capable of bonding with proteins, by inverse head polymerisation of a monomer phase which contains as monomer

1) 30 to 95 % by weight (based on the total weight of the polymerisable monomers) of at least one compound selected from the group comprising acrylamide, methacrylamide, methylene-bis-acrylamide and -methacrylamide,

2) optionally other hydrophilic radically polymerisable comonomers,

3) 5 to 60 % by weight of at least one unsaturated radically polymerisable monomer with an oxiran group,

4) optionally up to 25 % by weight of other radically polymerisable comonomers of low hydrophily, including at least 5 % by weight of monomer with at least two polymerisable double bonds and a diluent and is distributed in the form of droplets in a non-aqueous organic medium and is radically polymerised in this form, characterised in that the diluent used for the monomer phase is a mixture of

A) at least one solvent selected from the group comprising water, formamide, glycol and dimethylsulphoxide and

B) at least one organic liquid which is different from component A and which forms a homogeneous phase with the mixture of the monomers and component A and has a molecular weight of less than 200 and contains at least 20 % by weight of oxygen.

2. Process as claimed in claim 1, characterised in that a liquid which contains oxygen in the form of carbonyl and/or hydroxyl groups in used as component B of the diluent.

3. Process as claimed in claims 1 and 2, characterised in that an organic liquid with a total solubility parameter of between 9 and 15 Hildebrand units or a dispersive solubility parameter of 7 to 9 Hildebrand units is used as component B of the diluent.

4. Process as claimed in claims 1 to 3, characterised in that a liquid which is completely watermiscible at ambient temperature is used as component B of the diluent.

5. Process as claimed in claims 1 to 4, characterised in that a mixture of water and methanol is used as diluent.

6. Process as claimed in claims 1 to 5, characterised in that a polymer is dissolved in the monomer phase before the start of polymerisation.

8

**Revendications**

1. Procédé de préparation d'un polymère de support en perles, réticulé, hydrophile, capable de fixer des protéines, par polymérisation en perles inverse d'une phase monomère, qui renferme, comme monomères

1) 30 à 95 % en poids (rapporté au poids total des monomères polymérisables) d'au moins un composé choisi parmi l'acrylamide, le méthacrylamide, le méthylène -bis-acrylamide et le méthylène -bis-méthacrylamide,

2) éventuellement d'autres comonomères hydrophiles susceptibles de subir une polymérisation radicalaire,

3) 5 à 60 % en poids d'au moins un monomère susceptible de subir une polymérisation radicalaire insaturé comportant un groupe époxy,

4) éventuellement jusqu'à 25 % en poids d'autres comonomères susceptibles de subir une polymérisation radicalaire peu hydrophiles,

et, parmi eux, au moins 5 % de monomères comportant au moins deux doubles liaisons polymérisables, ainsi qu'un diluant, et qui est disséminée en gouttelettes dans un milieu organique non aqueux et subit une polymérisation radicalaire dans cette forme, caractérisé en ce qu'on utilise, comme diluant pour la phase monomère, un mélange comprenant

A) au moins un solvant choisi parmi l'eau, le formamide, le glycol et le diméthylsulfoxyde, et

B) au moins un liquide organique qui en diffère, formant avec le mélange des monomères et du constituant A une phase homogène, présentant un poids moléculaire inférieur à et renfermant au moins 20 % en poids d'oxygène.

2. Procédé selon la revendication 1, caractérisé en ce qu'on utilise, comme constituant B du du diluant, un liquide qui renferme l'oxygène sous forme de groupes carbonyle et/ou hydroxyle.

3. Procédé selon les revendications 1 et 2, caractérisé en ce que l'on utilise, comme constituant B du diluant, un liquide organique d'un paramètre de solubilité total compris entre 9 et 15 unités Hildebrand ou un paramètre de solubilité dispersive de 7 à 9 unités Hildebrand.

4. Procédé selon les revendications 1 à 3, caractérisé en ce qu'on utilise, comme constituant B du diluant, un liquide entièrement miscible avec l'eau à la température ambiante.

5. Procédé selon les revendications 1 à 4, caractérisé en ce qu'on utilise, comme diluant, un mélange d'eau et de méthanol.

6. Procédé selon les revendications 1 à 5, caractérisé en ce qu'on dissout un polymère dans la phase monomère avant le début de la polymérisation.